# EUROPEAN PATENT APPLICATION

(11) **EP 1 297 860 A1**
(43) Date of publication of application: **02.04.2003**
(21) Application number: 01938601.0
(22) Date of filing: 12.06.2001
(51) Int. Cl.: A61M 25/09, A61M 25/00, A61B 17/22, A61B 17/00

(54) **MEDICAL GUIDE WIRE DOUBLING AS CATHETER**

(30) Priority: 12.06.2000 JP 2000175993
(71) Applicant: ACP Japan Co. Ltd., Tokyo 113-0033 (JP)
(72) Inventor: SATA, Masataka, Toshima-ku, Tokyo 171-0021 (JP); NAKAMURA, Shoichi, Iruma-gun, Saitama 354-0041 (JP)
(74) Representative: Böckelen, Rainer
(86) International application number: JP0104940
(87) International publication number: WO01095975

(57) **Abstract**

This application is to provide a medical guide wire serving also as a catheter which requires only single insertion operation, and nevertheless by which it is possible to apply a pressure to a specified part on the internal wall of a vessel, to thereby expand the narrowed lumen, or to cut the part for removal, as well as to apply a therapeutic agent to the part.

The medical guide wire is inserted into the vasculature of a human or animal body comprising a guide wire body, and a bulge body attached to the distal tip end of the guide wire body, with the guide wire body enclosing a pipe connected to the bulge body and the bulge body including one, or two or more medicinal outlets connected to the pipe, whereby it is possible to ablate or to dilate a stenotic lesion on a specified part of the inner wall of a vessel, as well as to allow a therapeutic agent to flow out through the medicinal outlets to be applied to the lesion, by placing the bulge body close to the specified part and by vibrating or moving the bulge body in contact with the lesion.

## Description

### Technical Field

This invention relates to a medical guide wire serving also as a catheter which, being inserted into a blood vessel of a human or animal body, dilates a stenotic lesion developed on the inner wall of the vessel, or removes the stenotic lesion, and also has a function to inject a medicine or a gene into the stenotic lesion.

### Background Art

With a recent tendency that peoples have taken more westernized food in their meals than ever, they have come to consume a larger amount of meat. As a result of this tendency, the patients with ischemic heart disease and those with arteriosclerosis obliterans both based on atherosclerotic lesions are increasing. The immediate cause of those diseases is mainly explained by the following sequential events: cholesterol or similar compounds deposit on the inner wall of the vasculature; a stenotic lesion develops on that wall; and normal blood flow through the affected vessel is disturbed.

The percutaneous angioplasty or a therapy for treating arterial diseases based on atherosclerosis comes to be widely used. This is a treatment method for relieving a stenotic coronary artery of its stenotic lesion by way of percutaneous approach without resorting to thoracotomy, and to recover thereby the blood flow through the coronary artery. More specifically, a puncture is made on a femoral artery, and a guide wire is inserted through the puncture into the artery until its distal tip reaches the entry of a target coronary artery, and then the tip is allowed to remain there. Next, a balloon-attached catheter is introduced until the balloon is guided to the stenotic lesion, and then the balloon is inflated to give a pressure to the lesion.

Another type of percutaneous coronary angioplasty is also employed: a catheter with a cutter kept within its cylindrical tip is introduced until its tip is inserted into a stenotic lesion of a vessel; the atheroma lesion is then cut with the cutter for removal; and fragments generated as a result of cutting are collected through the cylindrical tip. For the treatment of a redeveloped atheroma lesion of a coronary artery, which is unresponsive to percutaneous angioplasty, stent placement is achieved via percutaneous approach.

As seen above, to apply various treatments to a stenotic lesion developed in a coronary artery, it is necessary to firstly make a puncture on a femoral artery or the like, to insert a guide wire through the puncture into the artery until its distal tip reaches the entry of the target coronary artery, and to keep the tip end there. Next, a balloon-attached catheter is introduced over the previously inserted guide wire to reach the part where the stenotic lesion has been located. Then, the balloon is inflated to apply a pressure against the stenotic lesion. Alternatively, a catheter enclosing a cutter in its distal tip is introduced until the distal tip reaches the stenotic lesion, and then the lesion is cut with the cutter for removal.

Indeed, when an atheroma lesion of a coronary artery is removed by such a technique based on pressure application, ablation or section, the incidence of the cases with initial complications decreases, and the reduction is accelerated with the improvement of relevant devices such as guide wires, catheters, etc. However, according to recent data, of the patients who have received such angioplasty operations, 20 to 50% experience renewed development of the stenotic lesion within several months subsequent to the initial operation. Stent placement consists of introducing a mesh stent, instead of a balloon, through an affected coronary artery, thereby keeping the artery expanded. Introduction of this technique results in the reduced incidence of renewed narrowing of the affected artery. However, even with this technique, about 20% of the patients still experience the renewed development of the lesion.

Pathohistological pictures of such redeveloped stenotic (restenotic) lesions have been studied, and the mechanism responsible for restenosis has been increasingly clarified. Specifically, when angioplasty is applied to a vessel with a stenotic lesion, a vascular damage mainly represented by endothelial injury results, and as a consequence smooth muscle cells in the media proliferate to cause restenosis.

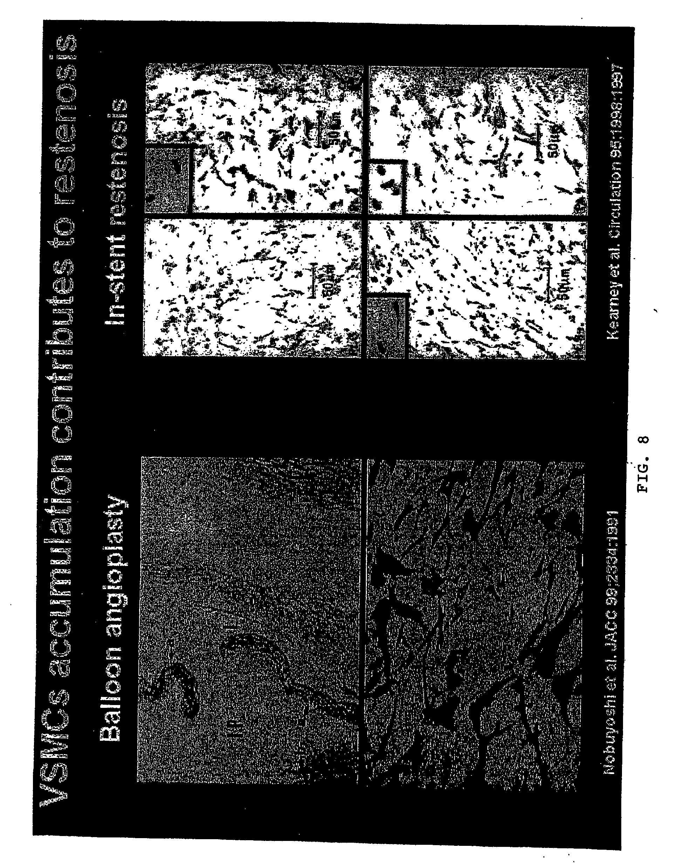

Fig. 8 shows pathological pictures of a restenotic lesion developed subsequent to angioplasty applied to a coronary artery. In Fig. 8 excess proliferation of smooth muscle cells is observed. Various medical therapies have been tried to prevent restenosis subsequent to coronary angioplasty. However, no drug has been found effective for the purpose in any large-scale clinical trials. Intra-coronary X-ray radiation has been also tried. However, this method is so problematic, because it may cause complications such as thrombosis, carcinogenesis, and proliferation of smooth muscle cells at the irradiated part, that it is hardly applicable to ordinary patients with coronary stenosis.

Fig. 9 shows damage associated with ballooning, the balloon being inserted into a mouse femoral artery. A puncture was made on a minute branch of the femoral artery, and a guide wire was inserted through the puncture into the femoral artery. The scale indicates 1 mm length.

Fig. 10 shows the pathological pictures of the mouse femoral artery showing the temporary change of the ballooning-associated damage. Immediately after ballooning, smooth muscle cells undergoing apoptosis in the media (TUNEL, positively stained cells) and the expanded intravascular lumen are observed. Then, smooth muscle cells proliferate excessively, and the intravascular lumen narrows.

Fig. 11 shows electronmicroscopic pictures of smooth muscle cells undergoing apoptosis. Clustering of chromatin particles is observed.

Fig. 12 shows the result of gene therapy introduced for the prevention of restenosis. When a gene responsible for the inhibition of cell cycling or p21 is introduced into a vessel suffering from a balloon-associated damage, proliferation of smooth muscle cells is suppressed.

As seen from above, as a method for treating an arterial disease, what might damage the artery in association with the treatment has been employed. One main complication associated with this method is restenosis due to the proliferation of smooth muscles cells. To avoid restenosis associated with such a treatment method, injection of a liquid medicine (therapeutic agent) or of a gene-based agent into the stenotic lesion has been tried.

It has been also tried to apply those therapies to mice using a tiny vessel of the mouse body to see whether they are effective, and to apply the result to human patients using a tiny vessel (of the brain, finger or pericardium) of them, and to establish those therapies based on the study results.

### Disclosure of Invention

However, the mouse vasculature is minute in size, for example, the femoral artery has a diameter of 0.2 to 0.3 mm. This makes it extremely difficult to apply ballooning to the mouse vasculature, a technique usually applied to large experimental animals such as rabbits and pigs. Specifically, it has been impossible to firstly insert a guide wire into a vessel of the mouse, and then to introduce a balloon catheter, whose size is barely sufficiently large to cover the guide tube, over the guide wire to effect ballooning, because the mouse vasculature is too minute for this procedure.

With regard to the animal study, to ensure its reliability, it is necessary to repeat the same experiment on many different animals. In this particular example, this means duplicate insertion of a guide wire and a catheter must be carried out for each experimental animal, and the same operation repeated for all the experimental animals in a comparatively short time. This may seriously impede the smooth progression of the experiment.

Let's consider how the above conventional technique will be conducted in a human patient. To dilate the narrowed part of a vessel such as a coronary artery or to remove cholesterol and tissue matrix depositing on the narrowed part, firstly a puncture is made on a femoral artery or a similar artery; a guide wire is inserted through the puncture into the artery until its distal tip reaches the target stenotic lesion; and the tip is kept there. Next, a balloon-attached catheter is passed over the previously inserted guide wire as far as the stenotic lesion. Then, a pressure is applied through the inflated balloon to the stenotic lesion. Alternatively, a catheter enclosing a cutter within its cylindrical tip is introduced into the stenotic lesion developed in the intravascular space, and the lesion is sectioned with the cutter for removal. Thus, for removal of each lesion, duplicate insertion of a guide wire and a balloon catheter must be performed.

This invention was proposed with a view to solve the aforementioned problem inherent to the conventional technique, and aims at providing a medical guide wire serving also as a catheter which requires only single insertion operation, and nevertheless by which it is possible to apply a pressure to a target stenotic lesion developed on the internal wall of a vessel, to thereby expand the narrowed lumen, or to cut the stenotic lesion for removal, as well as to apply a liquid medicine, a fluid chemotherapeutic agent or a gene-based agent (to be referred to together as a therapeutic agent hereinafter) to the lesion.

To attain this object, this invention provides a medical guide wire serving also as a catheter to be inserted into the vasculature of a human or animal body comprising a guide wire body, and a bulge body attached to the distal tip end of the guide wire body, with the guide wire body enclosing a pipe connected to the bulge body and the bulge body including one, or two or more medicinal outlets connected to the pipe, whereby it is possible to ablate or to dilate a stenotic lesion on a specified part of the inner wall of a vessel, as well as to allow a therapeutic agent to flow out through the medicinal outlets to be applied to the lesion, by placing the bulge body close to the specified part and by vibrating or moving the bulge body in contact with the lesion. The guide wire configured as above, once it is placed close to a specified part on the inner wall of a vessel, makes it possible to ablate the tissue there to cause stenosis to develop, or to dilate or ablate an existent stenotic lesion there, thereby intentionally giving a chance for restenosis to develop there.

The guide wire body has a coating layer formed on its surface, the coating layer being formed from a material comprising a water-soluble polymer substance or its derivative, and thus when the guide wire is brought into contact with an aqueous liquid, its surface turns to a low friction state, which makes it possible for the guide wire to be smoothly inserted into a blood vessel.

The bulge body has an electrode made from a conductive material to which is a lead wire or a conductive material is connected for voltage application.

This invention further provides a medical guide wire serving also as a catheter to be inserted into the vasculature of a human or animal body comprising a guide wire body, and a bulge body attached to the distal tip end of the guide wire body, with the guide wire body enclosing a pipe connected to the bulge body, and the bulge body comprising a first and second bulge bodies both made of a conductive material, and one, or two or more medicinal outlets connected to the pipe being implemented between the first and second bulge bodies, wherein a first potential source is connected to the first bulge body, and a second potential source is connected to the second bulge body, and ablation or dilatation of a stenotic lesion at a specified part within a vessel is achieved by vibrating or moving the bulge body placed at the specified part, and by applying, while a therapeutic agent is being flowed out from the medicinal outlets, a voltage or a ultrasonic vibration across the first and second bulge bodies.

Both the first and second bulge bodies are disc-shaped, and the voltage to be applied is a DC voltage of 0.1 to 10V.

### Brief Description of the Drawings

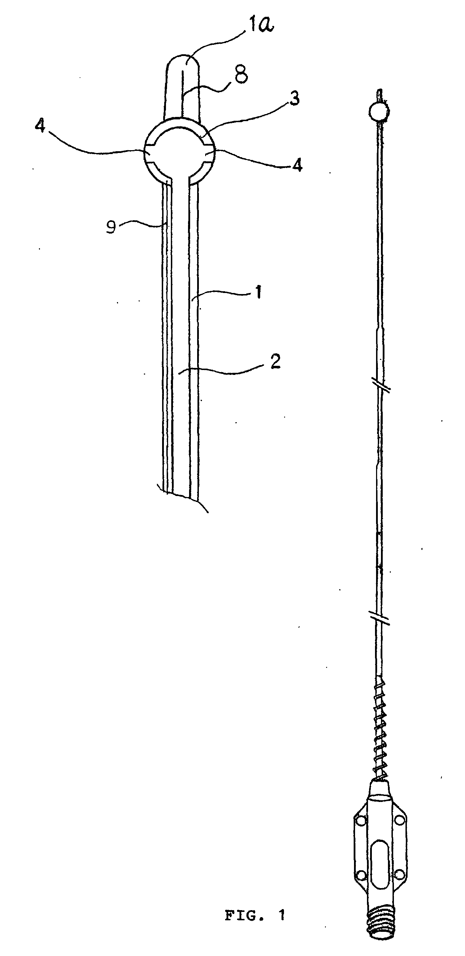

Fig. 1 shows an embodiment of a medical guide wire serving also as a catheter according to the present invention.

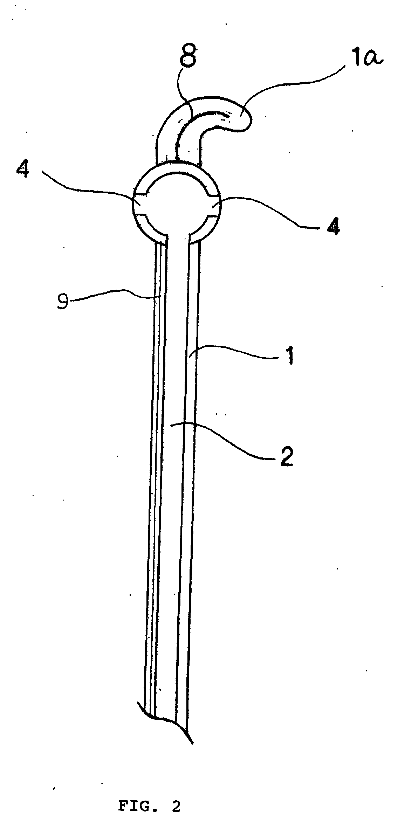

Fig. 2 shows a guide wire, which has its distal tip end smoothly, bent according to the present invention.

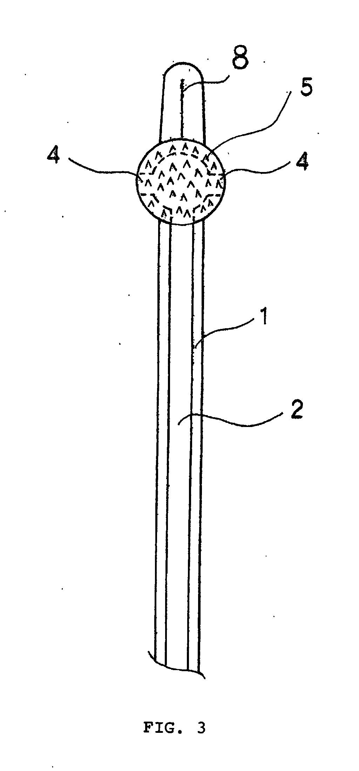

Fig. 3 shows a bulge body carrying tiny spines on its surface according to the present invention.

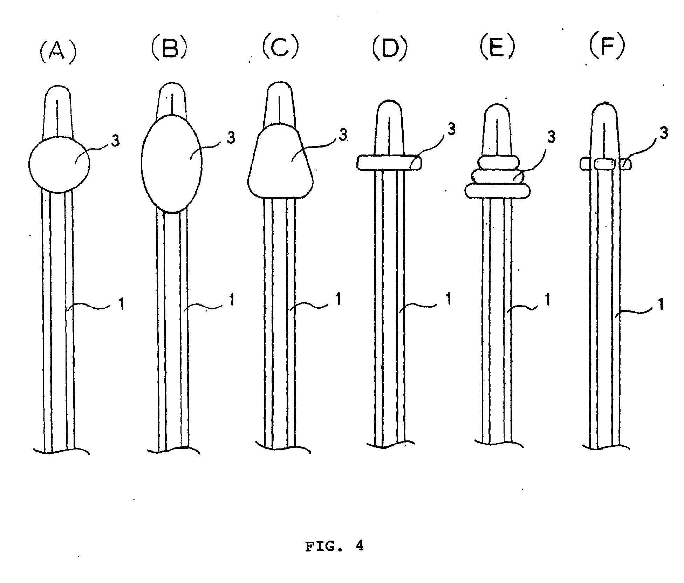

Fig. 4 shows various types of bulge bodies different in their shape prepared according to the present invention.

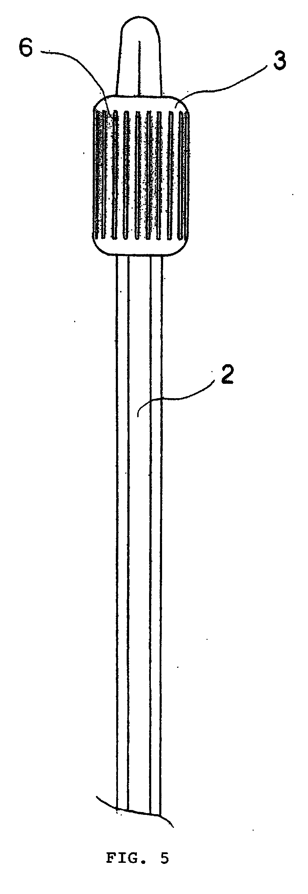

Fig. 5 shows a bulge body carrying slits on its surface according to the present invention.

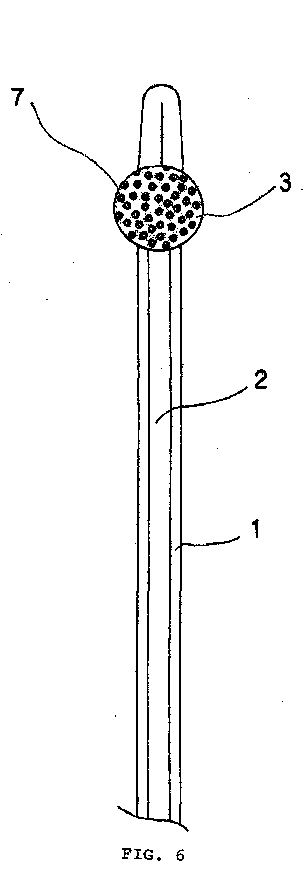

Fig. 6 shows a porous bulge body carrying many pores on its surface according to this invention.

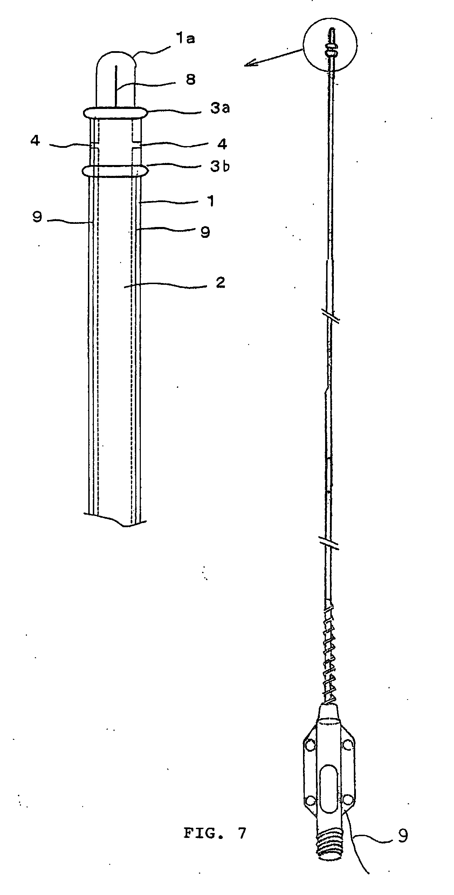

Fig. 7 shows a medical guide wire serving also as a catheter according to the present invention, and its bulge body consisting of two metal discs.
Fig. 8 shows the pathological pictures of a restenotic lesion subsequent to coronary angioplasty.

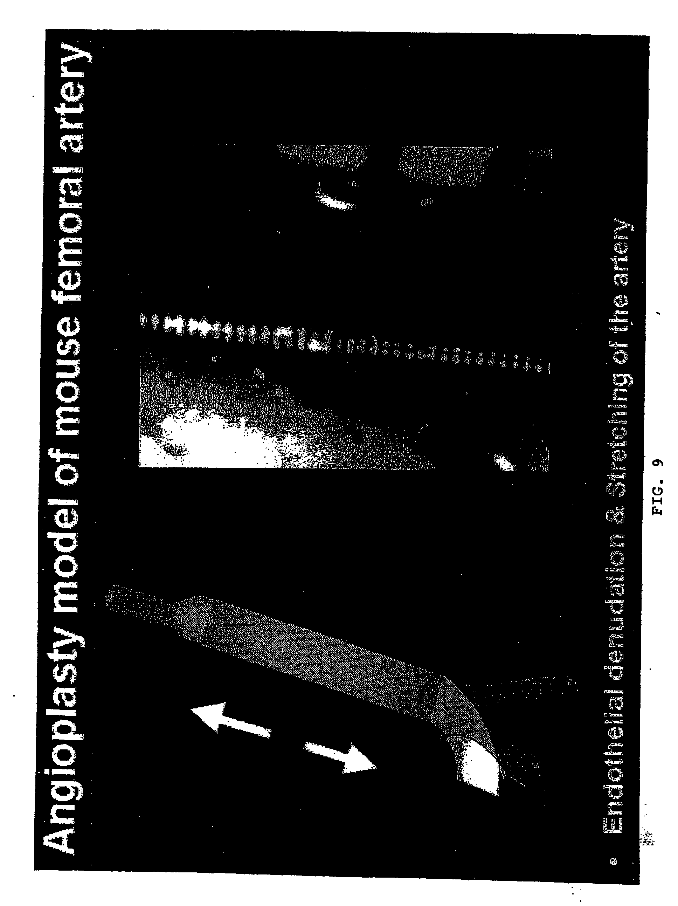

Fig. 9 shows an example of a damage inflicted on the mouse femoral artery as a result of ballooning.

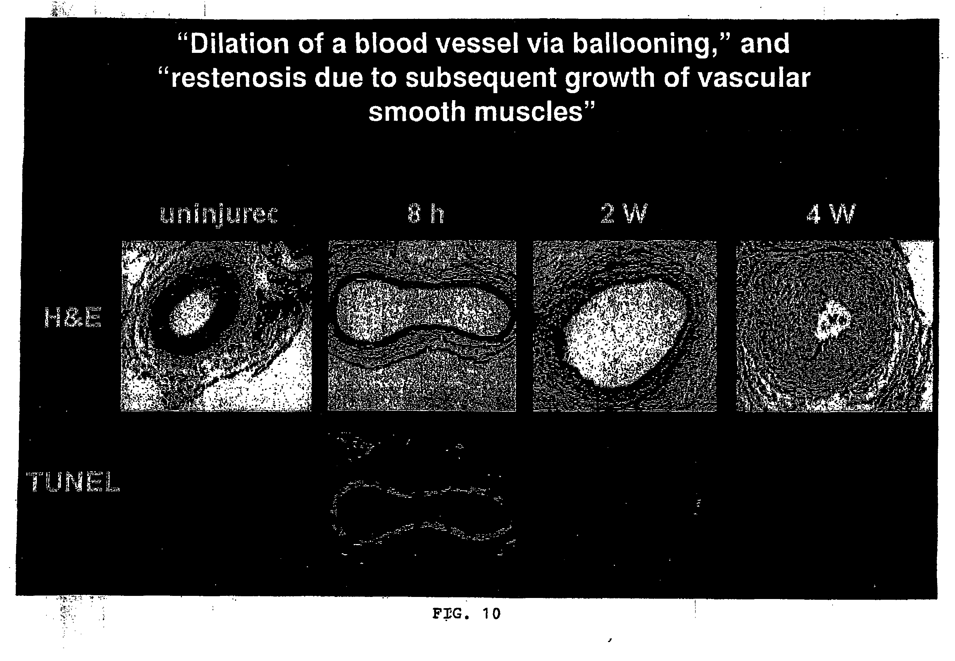

Fig. 10 shows the histological pictures of the damage of the mouse femoral artery subsequent to ballooning.

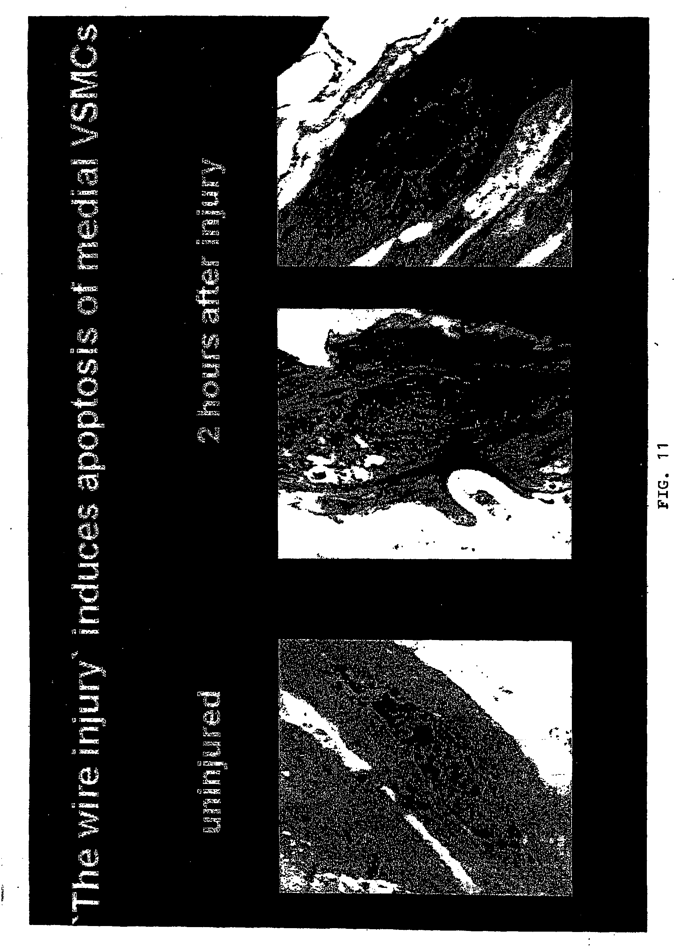

Fig. 11 shows an example of apoptosis of smooth muscle cells observed by electronmicroscopy.

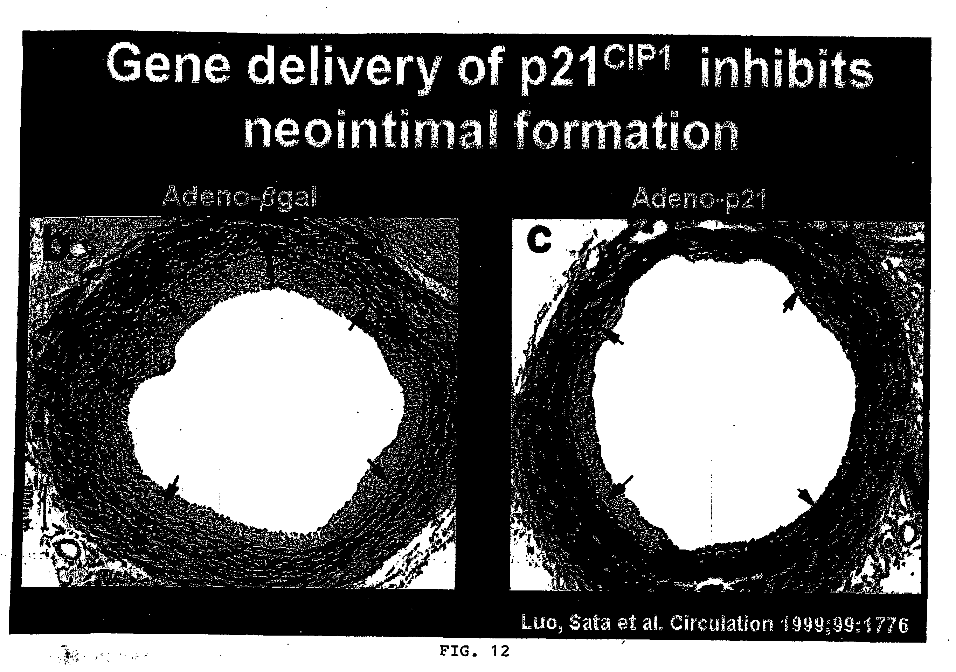

Fig. 12 shows the result of a gene therapy introduced for the prevention of restenosis.

### The Best Mode for Carrying out the Invention

The medical guide wire serving also as a catheter according to this invention will be described in detail below with reference to the attached figures.

Fig. 1 illustrates the important elements of the present invention. A wire member of the guide wire body 1 is made of a flexible single fiber, stainless steel fiber, piano fiber, titanium fiber or titanium alloy fiber.

The guide wire body 1 encloses, in its interior, a pipe 2 capable of passing a liquid therapeutic agent or a gene-based therapeutic agent, which may be introduced for preventing restenosis caused, for example, by proliferating smooth muscle cells. The therapeutic agent is supplied from the proximal end of the guide wire, and transported to a bulge body attached to the distal tip end of the guide wire under a predetermined pressure.

The guide wire body 1 may further include a core wire 8 having an appropriate stiffness in parallel with the pipe 2 as needed. This arrangement will make it possible for the guide wire 1 to smoothly take a desired branch at a bifurcation, given the flexibility of the wire member of the guide wire body. The core wire is made of a single fiber-like plastic member, stainless steel fiber, piano fiber, titanium fiber, titanium alloy fiber material or shape memory metal member. The core wire may be implanted in the guide wire body 1, at least in its tip end distal to the bulge body.

The bulge body 3 made from plastic or from a conductive material (metal member or the like) is attached close to the distal tip end 1a of the guide wire body 1. The bulge body 3 has openings 4 on its surface, so as to allow a therapeutic agent flowing through the pipe 2 to go outside.

The bulge body 3 made from a conductive material is further provided with an electrode connected to a lead 9 which will serve as an anode or a cathode. In this case, the human body serves as the opposite electrode. The lead 9, which is for applying a voltage to the bulge body 3, is implanted in the guide wire body 1, and the proximal end of the lead 9 coming out of the guide wire body 1 is connected to an external power supply (not illustrated here). The voltage to be applied to the bulge body 3 is a DC voltage of 0.1 to 10V, and the current passed is at a microampere level. It is possible, by introducing the wire body 1 to a specified part, to urge cells there to intake a gene or the like, by applying a predetermined voltage to the cells via the electrode.

Fig. 2 shows the distal tip end 1a of the wire body 1 which is bent to take a smoothly curved shape. The distal tip end 1a of the wire body 1 is bent into such a smoothly curved shape, so as to lessen the frictional resistance the wire body 1 will experience while it advances to a specified part. In addition, the wire body 1 has its entire surface coated with a known water-soluble polymer substance, such that the wire body 1 becomes highly affinitive to water, and thus, when it is brought into contact with an aqueous solution, it presents with a low frictional resistance to the solution.

The water-soluble polymer substance may include many known substances, natural and synthetic. The natural water-soluble polymer substance may include starch-based ones; cellulose-based ones; tannin and lignin-based ones; polysaccharides; and proteins such as gelatin, casein, etc. The synthetic water-soluble polymer substance may include PVA-based ones; polyethylene oxides; acrylates; anhydrous maleates; phthalates; water-soluble polyester and ketone aldehyde resins; acrylamide-based polymers; polyvinyl pyrrolidone; polyimine; and polyelectrolytes.

The guide wire body 1 is inserted from the inguinal part of a human or animal body, which requires treatment, until the guide wire 1 reaches a target part. Then, a therapeutic agent passing through the pipe 2 is flowed out from the openings 4 into the intravascular space.

Fig. 3 shows a bulge body having tiny spines formed on its surface. Because the bulge body 3 carries many tiny spines or projections 5 on its surface, it is possible to efficiently dilate or remove a stenotic lesion by bringing the bulge body 3 in contact with the lesion, while the bulge body is rotating.

Fig. 4 shows various types of bulge bodies different in their shape: type (A) has a round shape; type (B) oval one; type (C) pear-shaped one with a slender tip and thick base; and type (D) flat disc one; and type (E) consists of a row of disc plates each having a different diameter; and type (F) of a series of projections. From those types of bulge bodies 3, an appropriate one may be chosen depending on the geometry of a given target part.

Fig. 5 shows a bulge body having slits 6 on its surface. Specifically, the bulge body 3 has slits 6 on its surface, instead of dot-like openings 4, which allow a therapeutic agent to be injected into the intravascular space. When the bulge body 3 is introduced into a vessel to reach a target stenotic lesion, it is possible to ablate the lesion by rotating the bulge body 3, and then to recover the resulting fragments of the lesion through the slits 6 for disposal.

Fig. 6 shows a porous bulge body having many pores on its surface. The bulge body 3 has multiple tiny pores 7 on its surface, and a therapeutic agent supplied through the pipe 2 can be injected from those pores into the intravascular space. The bulge body may have multiple slits instead of pores, and whether slit-like or porous openings should be chosen must be determined according to the given purpose.

Another embodiment of the present invention will be described.

Fig. 7 illustrates the main components of another embodiment of this invention. The guide wire 1 encloses a pipe 2 in its interior through which a liquid agent or gene-based therapeutic agent can be transported. A first bulge body 3a and a second bulge body 3b both being formed from a conductive material are attached to the guide wire body 1 close to its distal tip end 1a. In this particular embodiment, both the first and second bulge bodies 3a and 3b are made of metal discs. The segment of the guide wire 1 between the first and second discs 3a and 3b includes one, or two or more openings 4 through which a therapeutic agent passing through the pipe 2 can be discharged into the intravascular space.

The two discs 3a and 3b are connected to respective conductive leads 9. The leads 9 for delivering a voltage across the two discs 3a and 3b are embedded in the guide wire body 1, and the proximal ends of the leads 9 coming out from the guide wire body 1 are connected to an external power source (not illustrated).

For treatment, the bulge body is placed in contact with a target stenotic lesion in the intravascular space, and its two disc plates 3a and 3b are allowed to vibrate or move to thereby ablate or dilate the stenotic lesion, and while a therapeutic agent (medicine) is released from the outlets 4, a voltage is applied across the first and second discs 3a and 3b. The outlets 4 are also utilized, once application of the therapeutic agent is completed, for recovering the fragments of the stenotic lesion ablated as above.

The voltage to be applied between the two disc plates 3a, 3b of the bulge body is a DC voltage of 0.1 to 10V. The current is in the range of microamperes.

Of the two disc plates 3a, 3b constituting the bulge body 3, one serves as a positive electrode while the other as a negative electrode. Alternatively, each of the electrodes can serve as a monopolar electrode.

For treatment, the bulge body 3 is placed at a specified part on the inner wall of a vessel; a stenotic lesion is ablated for removal or the stenotic lesion developed on the inner wall is dilated; and a gene or a therapeutic agent is flowed out from the outlets 4. During this process, it is possible to urge the cells to uptake the gene or the therapeutic agent by applying a predetermined voltage at the specified part. As a result of this therapeutic procedure, it is possible to reduce the period necessary for the resulting wound to heal, and to suppress the reformation of the lesion.

It was mentioned above that, of the two disc plates 3a, 3b constituting the bulge body 3, one serves as a positive electrode while the other as a negative electrode. However, one of the two electrodes may serve as a positive electrode while the human body serve as the ground electrode, making unnecessary the use of the other electrode.

As described above, the medical guide wire serving also as a catheter according to this invention allows one to, after single insertion operation, dilate a target stenotic lesion developed on the inner wall of a vessel by giving a pressure thereto or ablate it, and to administer a therapeutic agent to the lesion. With the medical guide wire serving also as a catheter, it is also possible to use it as a conductive material or a vibrating element, provided that the inner wall of the pipe 2 is insulated.

Specifically, in contrast with angioplasty based on the conventional catheter which requires firstly the insertion of a guide wire for guidance, then the insertion of a balloon-attached catheter for dilating a stenotic lesion, or of a catheter for removing cholesterol and tissue matrix depositing on the stenotic part, and lastly the insertion of a catheter for applying a therapeutic agent such as a medicine, or a gene or oligonucleotide to the lesion for inhibiting the growth of smooth muscles cells which would otherwise result to cause restenosis, angioplasty based on the medical guide wire serving also as a catheter according to this invention allows one to achieve the above three different operations almost simultaneously after a single insertion operation, and thus to achieve the operations in a shorter period.

### Industrial Applicability

The present invention is applicable for the production of a medical guide wire serving also as a catheter which, being inserted into a blood vessel of a human or animal body, can dilate a stenotic lesion developed on the inner wall of the vessel or ablate the lesion, and apply a medicine or a gene to the lesion.

## Claims

1. A medical guide wire serving also as a catheter to be inserted into a blood vessel of a human or animal body comprising a guide wire body, and a bulge body attached to the guide wire body close to its distal tip end, the guide wire enclosing a pipe passing through its interior to be connected to the bulge body and the bulge body having one, or two or more medicinal outlets communicating with the pipe,
whereby it is possible to produce an ablation at a specified part on the inner wall of the vessel or to dilate a stenotic lesion developed at the specified part by vibrating or moving the bulge body placed in contact with the lesion, and to allow a therapeutic agent to flow out from the medicinal outlets of the bulge body to be applied to the lesion.

2. A medical guide wire serving also as a catheter as described in Claim 1 wherein the guide wire has on its surface a coating layer made from a water-soluble polymer substance or its derivative, so that, when the guide wire is brought into contact with an aqueous solution, its surface turns to a low friction state.

3. A medical guide wire serving also as a catheter as described in Claim 2 wherein the guide wire from a part where the bulge body resides to its distal tip end includes a core wire, wherein the core wire is a coil member wound around an axis into a helix, and is made of a stainless steel wire, piano wire, titanium wire, or titanium alloy wire material.

4. A medical guide wire serving also as a catheter as described in Claim 2 wherein the water-soluble polymer substance or its derivative comprises a cellulose-, anhydrous maleate-, or acrylamide-based polymer substance.

5. A medical guide wire serving also as a catheter as described in Claim 1 wherein the bulge body is formed from an electrode composed of a conductive material to which is connected a lead for voltage application.

6. A medical guide wire serving also as a catheter as described in Claim 5 wherein the guide wire body encloses a lead or a conductive material through which it is possible to apply voltage to the bulge body.

7. A medical guide wire serving also as a catheter as described in Claim 1 wherein the bulge body is spherical in shape.

8. A medical guide wire serving also as a catheter as described in Claim 1 wherein the bulge body is oval in shape.

9. A medical guide wire serving also as a catheter as described in any Claim 1 to 3 wherein the bulge body is conical in shape with its distal end being slender and its proximal end being thick.

10. A medical guide wire serving also as a catheter as described in Claim 1 wherein the bulge body comprises one, or a row of two or more disc plates each having a different diameter.

11. A medical guide wire serving also as a catheter as described in Claim 1 wherein the bulge body has one, or two or more projections each having a different height.

12. A medical guide wire serving also as a catheter as described in any Claim 7 to 11 wherein the bulge body has fine spines or noduli on its surface.

13. A medical guide wire serving also as a catheter as described in Claim 3 wherein the distal tip end of the guide wire body takes a smooth streamline shape.

14. A medical guide wire serving also as a catheter as described in Claim 13 wherein, with regard to the bulge body, its portion close to the distal tip end is more slender and more flexible than the remaining portion.

15. A medical guide wire serving also as a catheter as described in Claim 3 wherein the medicinal outlets provided to the bulge body for releasing a liquid take the form of one, or two or more slit-like openings.

16. A medical guide wire serving also as a catheter as described in Claim 3 wherein the medicinal outlets provided to the bulge body take the form of porous openings.

17. A medical guide wire serving also as a catheter to be inserted into a blood vessel of a human or animal body comprising a guide wire body, and a bulge body attached to the guide wire body close to its distal tip end, the guide wire enclosing a pipe passing through its interior, and the bulge body being constituted of a first and second bulge bodies with one, or two or more medicinal outlets communicating with the pipe being implemented between the first and second bulge bodies, and a first potential source being connected to the first bulge body and a second potential source to the second bulge body,
whereby it is possible to produce an ablation at a specified part on the inner wall of the vessel or to dilate a stenotic lesion developed on the part by vibrating or moving the bulge body placed in contact with the lesion, and to apply a voltage or a ultrasonic vibration between the first and second bulge bodies, while a therapeutic agent is being released from the medicinal outlets to the lesion.

18. A medical guide wire serving also as a catheter as described in Claim 17 wherein the first and second bulge bodies are disc-like in shape.

19. A medical guide wire serving also as a catheter as described in Claim 17 wherein the voltage to be applied between the two bulge bodies is a DC voltage of 0.1 to 10V.

20. A medical guide wire serving also as a catheter as described in Claim 17 wherein the guide wire body encloses a lead or a conductive material so that voltage can be applied to the bulge body.
